Europäisches Patentamt

European Patent Office

Office européen des brevets

(19)

(11) Veröffentlichungsnummer: **0 1 1 1 808**
**B1**

(12) **EUROPÄISCHE PATENTSCHRIFT**

(45) Veröffentlichungstag der Patentschrift:
26.02.86

(21) Anmeldenummer: 83112195.9

(22) Anmeldetag: 05.12.83

(51) Int. Cl.⁴: **B 01 J 29/28**, C 07 C 5/27,
C 07 C 11/02

---

(54) **Neuer Katalysator, ein Verfahren zu seiner Herstellung und ein Isomerisierungsverfahren in Gegenwart dieses Katalysators.**

---

(30) Priorität: **16.12.82 DE 3246495**

(43) Veröffentlichungstag der Anmeldung:
**27.06.84 Patentblatt 84/26**

(45) Bekanntmachung des Hinweises auf die Patenterteilung:
**26.02.86 Patentblatt 86/9**

(84) Benannte Vertragsstaaten:
**DE FR GB NL**

(56) Entgegenhaltungen:
**EP - A - 0 035 807**
**EP - A - 0 051 318**
**US - A - 3 384 602**
**US - A - 3 660 513**
**US - A - 3 997 474**
**US - A - 4 101 598**
**US - A - 4 211 886**
**US - A - 4 324 698**

(73) Patentinhaber: **BAYER AG, Konzernverwaltung RP**
**Patentabteilung, D-5090 Leverkusen 1 Bayerwerk (DE)**

(72) Erfinder: **Schwerdtel, Wulf, Dr., Hegelstrasse 9,**
**D-5090 Leverkusen 1 (DE)**
Erfinder: **Lauer, Hubert, Dr., Iltisweg 8,**
**D-4047 Dormagen 11 (DE)**
Erfinder: **Heinrich, Josef, Dr., Berghausener Strasse 14,**
**D-4018 Langenfeld (DE)**

---

LIBER, STOCKHOLM 1986

## Beschreibung

Die vorliegende Erfindung betrifft einen neuen, aus bestimmten Alumosilikaten und bestimmten Tonmineralien herstellbaren Katalysator, ein Verfahren zu seiner Herstellung und ein Verfahren zur Skelettisomerisierung von n-Alkenen zu iso-Alkenen, das in Gegenwart dieses Katalysators durchgeführt wird.

Die Nachfrage nach iso-Alkenen, insbesondere solchen mit 4 und 5 C-Atomen, ist in letzter Zeit in stetigem Ansteigen begriffen. Beispielsweise werden größere Mengen an Isobuten zur Herstellung von Methyl-tert.butylether benötigt, dessen Bedeutung als bleifreier Kraftstoffverbesserer laufend zunimmt.

Es sind schon Verfahren zur katalytischen Skelettisomerisierung von n-Alkenen zu iso-Alkenen bekannt, die jedoch alle nachteilig sind, weil die bisher bekannten Katalysatoren kurze Standzeiten aufweisen und häufig regeneriert werden müssen. Als Katalysatoren sind beispielsweise aktivierte Aluminiumoxide wie Eta- oder Gamma-Al$_2$O$_3$, halogenierte Aluminiumoxide, Bauxite, mit Verbinminiumoxide, phosphate und feste phosphorsäuren bekannt.

So wird in der GB-A 20 60 424 ein Isomerisierungskatalysator zur Umwandlung von linearen aliphatischen Olefinen in verzweigte aliphatische Olefine beschrieben, der aktives Aluminiumoxid und darauf niedergeschlagen Fluor und Chlor enthält. Gemäß den Beispielen beträgt die Reaktionszeit an diesem Katalysator 1 oder 2 Stunden. In der DE-A 31 18 199 wird eine Skelettisomerisierung beschrieben, bei der ein fluoriertes Aluminiumoxid als Katalysator eingesetzt und in Gegenwart bestimmter Mengen Wasserdampf gearbeitet wird. Zu diesen Katalysatoren wird in der DE-A 30 00 650 ausgeführt, daß durch die Wasserdampfzugabe Fluor aus dem Katalysator ausgetragen und der Katalysator sehr schnell desaktiviert wird. Es wird vorgeschlagen, zur Aufrechterhaltung der Katalysatoraktivität Fluorverbindungen nachzudosieren. Dadurch kann die Zeit zwischen zwei Katalysatorregenerierungen auf ca. 50 Stunden angehoben werden, was aber immer noch sehr unbefriedigend ist. In der DE-A 25 34 459 wird ein Verfahren zur Isomerisierung von Alkenen beschrieben, bei dem ein Katalysator eingesetzt wird, der durch Behandeln von aktivem Aluminiumoxid mit bestimmten niedermolekularen Siliciumverbindungen hergestellt wird. Dieser Katalysator muß bereits nach 15 bis 20 Stunden regeneriert werden.

In der Ep-A 35 807 werden katalytische Umsetzungen an aus sogenanntem Silicalit bestehenden Katalysatoren beschrieben. Silicalit ist ein Mitglied der pentasil-Familie (Definition der pentasil-Familie siehe W.M. Meyer, G.T. Kokotailo "The propertieb and Applicntiona of Zeolites", Seite 133, The Chemical-Scriety London 1979), von denen eine Vielzahl bekannt sind, beispielsweise die ZSM-Typen und die Nu-Typen und ein bestimmtes kristallines Siliciumdioxid, das geringe Mengen Aluminiumoxid enthalten kann (s. DE-A 27 51 443). Gemäß den Angaben in der Ep-A 35 807 fällt der Silicalit bei seiner Herstellung in Form eines feinen pulvers an. Wenn es als stückiger Katalysator eingesetzt werden soll, kann es mit einem Bindemittel, z.B. Kaolin, Verbetzt und Verformt Herden. Kaolin kann dabei aber nur dann als Bindemittel wirken, wenn das Gemisch aus Silicalit und Kaolin auf Temperaturen über 550°C erhitzt wird, dh Kaolin sich erst über 550°C in Metakaolin verwandelt (s. Ullman, Enzyklopkdie der technischen Chemie, 4. Aufinge, Band 17, Beite 587).

Der EP-A 35 807 kann nicht entnommen werden, daß Mischungen aus kristalliaiertem Aluminiumsilikat und Kaolinit enthaltenden Tonminbralien, die bei max. 550°C, also deutlich unterhalb der Abbindetemperatur von Kaolin, calciniert werden, vorteilhafte Katalysatoren ergeben könnten.

Es wurde nunmehr ein Katalysator gefunden, der dadurch gekannzeichnet ist, daß er erhältlich ist, indem man kristallisihrtes Alumosilikat, das 0,05 bis 2 Gew.-% Fluor enthält und das ein Molverhältnis von Silicium zu Aluminium von 100:1 bis 10 000:1 aufweist, mit einem Kaolinit enthaltenden Tanmineral mischt, das mit der Lösung eines oder mehrerer Alkaliund/oder Erdalkalisalze vorbehandelt wurde, gegebenenfalls das Gemisch zusätzlich mit Bindemitteln und/oder Füllstoffen versetzt und verformt, und bei 350 bis 550° C calciniert.

Als kristallisierte Alumosilikate kommen vorzugsweise Alumosilikate vom Zeolith-Typ zum Einsatz, insbesondere solche mit den Strukturmerkmalen der pentasil-Familie. Besonders bevorzugt sind kristallisierte Alumosilikate wie sie in der US-A 3 702 886 und der DE-A 27 51 443 beschrieben sind.

Das kristallisierte Alumosilikat weist vorzugsweise ein Molverhältnis von Silicium zu Aluminium von 300:1 bis 5000:1 auf.

Als Kaolinit enthaltende Tonmineralien kommen insbesondere Kaolin in Frage und Tonmineralien, die Kaolin enthalten. Beispielsweise sind Tonmineralien geeignet, die 20 bis 100 Gew.-% Kaolin enthalten.

Das Mischen von kristallisiertem Alumosilikat und Kaolinit enthaltendem Tonmineral kann auf übliche Weise durchgeführt werden. Wenn diese Ausgangsstoffe nicht bereits in feinpulvriger Form vorliegen, so werden sie vorteilhafterweise fein gemahlen, gegebenenfalls unter Zusatz von Hilfsstoffen, beispielsweise Wasser.

Wenn man die erfindungsgemäßen Katalysatoren in pulvriger Form erhalten will, so kann das Gemisch aus kristallisiertem Alumosilikat und Kaolinit enthaltendem Tonmineral ohne weitere Vorbehandlung bei 350 bis 550°C calciniert werden. Will man stückige Katalysatoren erhalten, so kann man das Gemisch aus kristallisiertem Alumosilikat und Kaolinit enthaltendem Tonmineral mit üblichen Bindemitteln und/oder üblichen Füllstoffen versetzen und verformen und erst danach bei 350 bis 550°C, vorzugsweise bei 450 bis 550°C, calcinieren. Im allgemeinen sind Zeiten im Bereich Von 1 bis 5 Stunden für die Calcinierung gusreichend. Das Gewichtsverhältnis von kristallisiertem Alumosilikat zu Kaolinit enthaltendem Tornmineral kann beispielsweise im Bereich von 0,4:1 bis 20:1 liegen. Vorzugsweise beträgt dieses Gewichtsverhältnis 1,5:1 bis 9:1.

Erfindungsgemäß wird ein kristallisiertes Alumosilikat einsetzt, das 0,05 bis 2 Gew.-% Fluor enthält. Man kann dabei so vorgehen, daß man das kristalline Alumosilikat vor dem Vermischen mit dem Kaolinit enthaltenden Tonmineral so lange mit einer Fluorverbindung behandelt, bis der Fluorgehalt 0,05 bis 2 Gew.-% betragt. Als

2

# 0 111 808

Fluorverbindungen eignen sich beispielsweise Fluorwasserstoff, Ammoniumfluorid, Fluorborsaure, Fluorsiliciumsäure und fluororganische Verbindungen. Die Behandlung kann beispielsweise so erfolgen, daß man das kristalline Alumosilikat mit einer Lösung einer Fluorverbindung imprägniert, z.B. mit einer wäßrigen Ammoniumfluoridlösung, und anschließend das so imprägnierte kristalline Alumosilikat trocknet und bei 450 bis 550°C calciniert.

Erfindungsgemäß wird weiterhin ein Koalinit enthaltendes Tonmaterial einsetzt, das mit der Lösung eines oder mehrerer Alkali- und/oder Erdalkalisalze vorbehandelt wurde. Diese Vorbehnndung kann beispielsweise so durchgeführt werden, daß man das Kaolinit enthaltende Tonmineral mit einem Überschuß einer wäßrigen Lösung eines wasserlöslichen Alkali- und/oder Erdalkalisalzes imprägniert, beispielsweise bei erhöhter Temperatur, und das Kaolinit enthaltende Tonmineral anschließend gründlich mit Wasser auswascht und trocknet.

Die vorliegende Erfindung betrifft weiterhin ein Verfahren zur Herstellung eines Katalysators, das dadurch gekennzeichnet ist, daß man kristallisiertes Alumosilikat, das 0,05 bis 2 Gew.-% Fluor enthält und das ein Molverhältnis von Silicium zu Aluminium von 100:1 bis 10 000:1 aufwest, mit einem Kaolinit enthaltenden Tonmineral mischt, das mit de Lösung eines oder mehrerer Alkali- und/oder Erdalkalisalze vorbehandelt wurde, gegebenenfalls das Gemisch zusätzllich mit Bindemitteln und/oder Füstoffen versetzt und verformt, und bei 350 bis 550° C calciniert.

Das Mischen von Alumnosilikat und Kaolinit enthaltendem Tonmineral kann auf beliebige Weise erfolgen, beispielsweise durch Mischung der trockenen oder, z.B. mit Wasser angefeuchteten, pulverförmigen Materialien. Wenn man die Ausgangsmaterialien als pulverförmige Stoffe ohne Zusatze von Bindemitteln und/oder Füllstoffen calciniert, so fallen die erfindungsgemäßen Katalysatoren in pulverform an. Zum Einsatz in Wirbelbettreaktoren können die erfindungsgemäßen Katalysatoren beispielsweise als Mikrogranulate hergestellt werden, z.B. nach an sich bekannten Sprühgranulations-Techniken. Man kann erfindungs-gemäße Katalysatoren auch in stückiger Form herstellen, die für den Einsatz als fest angeordnete Katalysatoren geeignet ist. Hierbei ist es erforderlich, dem Gemisch aus kristallisiertem Alumosilikat und Kaolinit enthaltendem Tonmineral Bindemittel und/oder Füllstoffe zuzusetzen und es anschließend zu verformen. Geeignete Bindemittel sind beispielsweise Kieselsol oder Kieselgel, geeignete Füllstoffe sind vorzugsweise inert, wie amorphes Siliciumdioxid. Die Verformung kann nach bekannten Methoden erfolgen, beispielsweise durch Extrudieren, Pelletisieren oder Verpressen. Wenn bei der Katalysatorherstellung Bindemittel und/oder Füllstoffe eingesetzt werden und eine Verformung durchgeführt wird, so ist es vorteilhaft, die nach dieser Behandlung vorliegenden Teilchen zu trocknen und erst dann bei Temperaturen von 350 bis 550°C zu calcinieren.

Die vorliegende Erfindung betrifft weiterhin ein Verfahren zur katalytischen Skelettisomerisierung von n-Alkenen zu iso-Alkenen, das dadurch gekennzeichnet ist, daß man die Isomerisierung in Gegenwart von Katalysatoren durchführt, die erhältlich sind, indem man kristallisiertes Alumosilikat, das ein Molverhältnis von Silicium zu Aluminium von 100:1 bis 10 000:1 aufweist, mit einem Kaolinit enthaltenden Tonmineral mischt, gegebenenfalls das Gemisch mit Bindemitteln und/oder Füllstoffen versetzt und verformt, und bei 350 bis 550°C calciniert.

In dem erfindungsgemäßen Verfahren zur katnlytihchen Skelettisonerisierung bzw. bei der Verwendung der erfindungsgemäßen Katalysatoren bei der Skelettibomerisierung sind hinsichtlich des Katalysators solche bevorzugt, bei denen das eingesetzte kristalline Alumosilikat 0,05 bis 2 Gew.-% Fluor enthält und bei denen dss eingesetzte Kaolinit enthaltende Tonmineral mit der Lösung eines oder mehrerer Alkali- und/oder Erdalkalissize vorbehandelt wurde.

Die erfindungsgemkgs katalytische Skelettisomerisierung wird im allgemeinen in der Gasphase bei Reaktionstemperaturen im Bereich 350 bis 550° C, vorzugsweise im Bereich 450 bis 550° C, durchgeführt. Der Katalysator kann dabei fest in einem Reaktor angeordnet sein, es kann aber auch in einem bewegten Bett, z.B. im Wirbelbett, gearbeitet werden.

Für die Durchführung der Isomerisierung im Wirbelbett sind pulverförmige Katalysatoren oder solche in Form von Mikrogranulaten geeignet, d.h. bei der Herstellung solcher Katalysatoren ist es, wie vorstehend beschrieben, nicht notwendig, das Gemisch aus kristallisiertem Alumosilikat und Kaolinit enthaltendem Tonmineral mit Bindemitteln und/oder Füstoffen zu versetzen und zu verformen.

Für die Durchführung der Isomerisierung mit fest angeordneten Katalysatoren sind stückige Katalysatoren geeignet, die wie vorstehend beschrieben hergestellt werden können.

Nach dem erfindungsgemäßen Verfahren können beliebige n-Alkene zu iso-Alkenen isomerisiert werden, beispielsweise solche mit 3 bis 10 C-Atomen. Dem erfindungsgemäßen Verfahren zur Skelettisomerisierung werden vorzugsweise n-Alkene mit 4 bis 6 Kohlenstofatomen unterworfen, besonders bevorzugt n-Alkene mit 4 Kohlenstoffatomen (n-Butene) und mit 5 Kohlenstoffatomen (n-Pentene). Die n-Alkene können in reiner Form, in Mischungen untereinander oder in Mischungen mit anderen Kohlenwasserstoffen, insbesondere den entsprechenden Alkanen, eingesetzt werden. Es kann vorteilhaft sein, die n-Alkene oder n-Alkene enthaltenden Einsatzgemische mit Gasen zu verdünnen. Hierfür sind beispielsweise geeignet:.

Stickstoff, Kohlendioxid und/oder Wasserdampf. Insbesondere Wasserdampf begünstigt die Reaktion in der Weise, daß die Katalysatoraktivität bei hoher Selektivität über sehr lange Betriebszeiten aufrechterhalten wird. Die Menge des zugegebenen Wasserdampfes kann in weiten Grenzen schwanken, besonders günstig ist ein Molverhältnis von Wasser zu eingesetzten Kohlenwasserstoffen von 0,1:1 bis 10:1.

Geeignete Betriebsdrucke für die erfindungsgemäße Skelettisomerisierung sind beispielsweise solche von 0,1 bis 10 bar. Drucke im Bereich von 1 bis 5 bar sind bevorzugt.

Die Raumdurchsatzgeschwindigkeit, ausgedrückt als WHSV (weight hourly space velocity), kann bei der

3

erfindungs-gemäßen Skelettisomerisierung beispielsweise im Bereich von 0,1 bis 20 liegen. Vorzugsweise beträgt die Raumdurchsatzgeschwindigkeit 0,2 bis 10 kg n-Alkene pro Liter Katysator und Stunde.

Die Verweilzeit der Einsatzmaterialien in der Reaktionszone beträgt beim erfindungagemäßen Verfahren vorzugsweise weniger als 5 Sekunden.

Wenn bei der erfindungsgemäßen katalytischen Skelettisomerisierung die Katalyshtoren nach längerer Betriebszeit in ihrer Aktivität nachlassen, können sie leicht nach bekannten Methoden regeneriert werden. Beispielsweise kann man dabei Luft oder Luft/Wasserdampf-Gemische bei Temperaturen von beispielsweise 400 bis 600° C über den Katalysator leiten.

Die erfindungsgemaßen Katalysatoren haben den Vorteil, daß sie beim Einsatz in die katalytische Skelettisomerisierung von n-Alkenen zu iso-Alkenen im Vergleich zu bekannten Katalysatoren sehr viel längere Betriebszeiten zulassen, bevor sie regeneriert werden müen. Die Betriebszeit der erfindungsgemäßen Katalysatoren liegt im allgemeinen bei mehreren 100 Stunden und kann bis über 1000 Stunden betragen.

Die erfindungsgemäßen Katalysatoren zeichnen sich außerdem durch besondere Aktivität und besonders gute Selektivität fü die Umwandlung von n-Alkenen zu iso-Alkenen aus.

Im Gegensatz zur Ep-A 35 807 Hirkt das Kaolinit enthaltende Tonmineral bei der vorliegendcn Erfindung nicht als Bindemittel, sondern stellt einen wesentlichen Teil des Katalysators dar. Der erfindungsgemaße Katalysator ist nur dann bei dcr Skelettisomerisierung wirksam, wenn das Alumosilikat und das Taolinit enthaltende Tonmineral gemeinsam vorliegen. Die Einzelkomponenten sind bei der Skelettisomerisierung nicht katalytisch wirksam. Ebenfalls bei der Skelettisomtrisierung katalytisch unwirksame Stoffe erhält man, wenn man Alumosilikat und Kaolinit enthaltende Tonmineralien bei Temperaturen calciniert, bei denen eine Umwandlung des Kaolinits in Metakaolin stattfindet (s. Beispiel 6).

Die folgenden Beispiele erläutern die Erfindung ohne sie in irgendeiner Weise zu beschränken.

## Beispiele

Definitionen von in den folgenden Beispielen verwendeten Begriffen:

### $C_4$-Einsatz:

Ein Kohlenwasserstoffgemisch der Zusammensetzung

| | |
|---|---|
| Isobutan | 6,90 Gew.-% |
| n-Butan | 22,80 Gew.-% |
| n-Buten-1 | 37,80 Gew.-% |
| i-Buten | 0,90 Gew.-% |
| t-Buten-2 | 11,20 Gew.-% |
| i-Buten-2 | 20,40 Gew.-% |

### WHSV:

weight hourly space velocity = kg Einsatzprodukt pro Liter Katalysator und Stunde.

### $C_{5+}$:

Produkte mit 5 und mehr Kohlenstoffatomen.

$\leqslant C_3$:

Produkte mit 1 bis 3 Kohlenstoffatomen.

**Umvandlung n-Butene (%):**

$$\frac{\left(\sum \% \text{ n-Butene}_{\text{Eingang}} - \sum \% \text{ n-Butene}_{\text{Ausgang}}\right) \cdot 100}{\sum \text{ n-Butene}_{\text{Eingang}}}$$

**Selektivität für Isobuten (%):**

$$\frac{\left(\% \text{ Isobuten}_{\text{Eingang}} - \% \text{ Isobuten}_{\text{Ausgang}}\right) \cdot 100}{\sum \% \text{ n-Butene}_{\text{Eingang}} - \sum \% \text{ n-Butene}_{\text{Ausgang}}}$$

**Selektivität für Propen (%):**

$$\frac{\% \text{ Propen} \cdot 100}{\sum \% \text{ n-Butene}_{\text{Eingang}} - \sum \% \text{ n-Butene}_{\text{Ausgang}}}$$

**Selektivität für $C_{5+}$ (%):**

$$\frac{\% \ C_{5+} \cdot 100}{\sum \% \ \text{n-Butene}_{\text{Eingang}} \ - \ \sum \% \ \text{n-Butene}_{\text{Ausgang}}}$$

**Selektivität für $\leqslant C_3$ (%):**

$$\frac{\% \ \leqslant C_3 \cdot 100}{\sum \% \ \text{n-Butene}_{\text{Eingang}} \ - \ \sum \% \ \text{n-Butene}_{\text{Ausgang}}}$$

**RZA für Isobuten:**

Raum/Zeit-Ausbeute = g Isobuten pro Liter Katalysator und Stunde.

**Beispiel 1**

In einem Rührautoklaven wurden 7 kg Wasser und 0,3 kg konzentrierte Natronlauge vorgelegt. In dieser Vorlage wurden 0,4 kg Tetrapropylammoniumbromid gelöst und 15 Minuten gerührt. Anschließend wurden unter Rühren 6 kg Kieselsol (30 %ig) so langsam zugegeben, daß eine homogene Mischung entstand. Bei 150°C wurde das entstandene Gel innerhalb von 5 Tagen kristallisiert. Das so erhaltene kristallisierte Alumosilikat wies die für pentai sile typischen röntgenographischen d-Werte auf und enthielt Silicium und Aluminium in einem Molverhältnis von 440:1.

50 g dieses Alumosilikats wurden mit 10 g Kaolin vom Typ Dorfner H 1 in 25 g Wasser aufgeschlämmt und vermischt. Das Gemisch wurde in eine Lochplatte gepreßt und getrocknet. Das so verformte produkt wurde dann 3 Stunden bei 450°C calciniert.

Der so hergestellte Katalysator wurde in einen elektrisch beheizten Röhrenreaktor mit einem Volumen von 30 ml eingebracht. Mittels mechanischer Mengenregler wurde $C_4$-Einsatz und Wasserdampf zudosiert. Die Reaktortemperatur lag bei 500°C. Der den Reaktor verlassende Gasstrom wurde gaschromatographisch untersucht. Dabei wurden die in Tabelle 1 angegebenen Ergebnisse erhalten.

## Tabelle 1

| Betriebsstunden | 12 | 24 | 36 | 48 | 60 | 72 | 84 | 96 |
|---|---|---|---|---|---|---|---|---|
| WHSV für $C_4$-Einsatz | 1,64 | 1,64 | 1,69 | 1,61 | 1,67 | 1,69 | 1,67 | 1,67 |
| WHSV für Wasserdampf | 0,5 | 0,5 | 0,5 | 0,5 | 0,5 | 0,5 | 0,5 | 0,5 |
| Isobutan (Gew.-%) | 6,56 | 5,57 | 4,69 | 7,78 | 7,47 | 6,60 | 5,90 | 5,07 |
| Propen (Gew.-%) | 1,23 | 1,27 | 1,33 | 1,30 | 1,28 | 1,29 | 1,21 | 1,20 |
| n-Butan (Gew.-%) | 22,65 | 22,83 | 22,75 | 23,02 | 23,20 | 22,96 | 23,13 | 23,15 |
| n-Buten-1 (Gew.-%) | 16,91 | 16,99 | 17,28 | 16,48 | 16,63 | 16,79 | 17,03 | 17,04 |
| 1-Buten (Gew.-%) | 8,42 | 8,16 | 8,21 | 8,10 | 7,81 | 7,55 | 7,69 | 7,87 |
| t-Buten-2 (Gew.-%) | 24,25 | 24,40 | 24,70 | 23,67 | 23,59 | 24,50 | 24,58 | 25,00 |
| 1-Buten-2 (Gew.-%) | 17,50 | 18,01 | 18,25 | 17,17 | 17,41 | 17,64 | 17,79 | 17,98 |
| $C_{5+}$ (Gew.-%) | 2,43 | 2,71 | 2,73 | 2,44 | 2,54 | 2,81 | 2,41 | 2,63 |
| $C_3$ (Gew.-%) | 0,05 | 0,05 | 0,06 | 0,04 | 0,06 | 0,05 | 0,06 | 0,06 |
| Umwandlung n-Butene (%) | 22,5 | 22,7 | 22,9 | 25,5 | 26,6 | 24,1 | 24,6 | 24,4 |
| Selektivität für Iso-buten (%) | 69,4 | 66,8 | 66,6 | 68,2 | 66,8 | 64,7 | 67,6 | 66,9 |
| Selektivität für Propen (%) | 10,2 | 10,4 | 10,8 | 10,9 | 11,0 | 11,0 | 10,6 | 10,2 |
| Selektivität für $C_{5+}$ (%) | 20,2 | 22,2 | 22,1 | 20,5 | 21,7 | 24,0 | 21,2 | 22,4 |
| RZA für Isobuten | 138,0 | 133,8 | 138,7 | 130,4 | 130,4 | 127,6 | 128,4 | 131,4 |

**Beispiel 2**

50 g des nach Beispiel 1 hergestellten kristallinen Alumosilikats wurden mit einer Lösung von 1 g Ammoniumfluorid in 50 ml Wasser aufgeschlämmt. Nach dem Trocknen und 3-stündigen Calcinieren bei 450°C enthielt das Material 0,35 Gew.-% Fluor. Dieses fluorhaltige kristalline Aluminiumsilikat wurde mit Kaolin vermischt, so daß das Gewichtsverhältnis von kristallinem Aluminiumsilikat zu Kaolin 4:1 betrug. Dieses Gemisch wurde analog Beispiel 1 mit Wasser versetzt und durch pressen in eine Lochplatte und Trocknen zu preßlingen verformt und dann 3 Stunden bei 450°C calciniert.

Der so hergestellte Katalysator wurde gemäß der im Beispiel 1 beschriebenen Verfahrensweise für die Umwandlung von n-Buten zu iso-Buten eingesetzt. Die dabei erhaltenen Ergebnisse sind in Tabelle 2 zusammengefaßt.

## Tabelle 2

| Betriebsstunden | 12 | 24 | 48 | 72 | 96 | 108 | 120 |
|---|---|---|---|---|---|---|---|
| WHSV für $C_4$-Einsatz | 1,67 | 1,72 | 1,53 | 1,75 | 1,61 | 1,64 | 1,58 |
| WHSV für Wasserdampf | 0,5 | 0,5 | 0,5 | 0,5 | 0,5 | 0,5 | 0,5 |
| Isobutan (Gew.-%) | 6,10 | 5,10 | 8,11 | 6,76 | 5,06 | 3,89 | 7,73 |
| Propen (Gew.-%) | 5,99 | 5,18 | 3,44 | 3,51 | 3,49 | 3,69 | 3,30 |
| n-Butan (Gew.-%) | 20,67 | 20,77 | 20,16 | 20,22 | 20,62 | 20,13 | 22,21 |
| n-Buten-1 (Gew.-%) | 12,42 | 13,34 | 13,20 | 14,13 | 14,72 | 14,92 | 12,97 |
| 1-Buten (Gew.-%) | 15,50 | 15,02 | 13,82 | 13,40 | 13,95 | 14,09 | 15,61 |
| t-Buten-2 (Gew.-%) | 18,03 | 19,14 | 19,26 | 20,94 | 21,31 | 21,60 | 19,23 |
| 1-Buten-2 (Gew.-%) | 13,30 | 14,19 | 14,55 | 15,38 | 15,69 | 15,94 | 13,94 |
| $C_{5+}$ (Gew.-%) | 7,54 | 6,89 | 7,14 | 5,13 | 4,94 | 5,50 | 4,60 |
| -$C_3$ (Gew.-%) | 0,4 | 0,37 | 0,32 | 0,53 | 0,22 | 0,24 | 0,41 |
| Umwandlung n-Butene (%) | 43,6 | 41,4 | 41,5 | 36,4 | 36,3 | 32,0 | 34,6 |
| Selektivität für Iso-buten (%) | 52,7 | 54,7 | 55,9 | 60,2 | 61,7 | 59,9 | 62,1 |
| Selektivität für Pro-pen (%) | 20,4 | 18,8 | 13,9 | 15,8 | 15,4 | 15,4 | 15,7 |
| Selektivität für $C_{5+}$ (%) | 25,6 | 25,1 | 28,9 | 23,0 | 21,8 | 23,4 | 21,3 |
| Selektivität für -$C_3$ (%) | 1,3 | 1,4 | 1,3 | 1,0 | 1,1 | 1,3 | 0,9 |
| RZA für Isobuten | 258,8 | 258,3 | 211,4 | 234,5 | 224,6 | 231,1 | 246,6 |

**Beispiel 3**

50 g des nach Beispiel 1 hergestellten kristallinen Alumosilikats wurden wie in Beispiel 2 beschrieben fluoriert. 20 g Kaolin wurden mit 300 ml wäßriger 2 n $MgCl_2$-Lösung 3 Stunden bei 100°C gerührt. Das so behandelte Kaolin wurde abfiltriert, mit Wasser gewaschen und getrocknet. Danach wurden 40 g des fluorierten Alumosilikats und 6 g des behandelten Kaolins vermischt, durch Verformung über eine Lochplatte in analoger Weise zu Beispiel 1 zu preßlingen verformt und anschließend 3 Stunden bei 450°C calciniert.

Der so hergestellte Katalysator wurde gemäß der in Beispiel 1 beschriebenen Verfahrensweise für die Umwandlung von n-Buten zu iso-Buten eingesetzt. Die dabei erhaltenen Ergebnisse sind in Tabelle 3 zusammengefaßt.

**Tabelle 3**

| Betriebsstunden | 12 | 24 | 48 | 96 | 144 | 192 | 240 | 288 |
|---|---|---|---|---|---|---|---|---|
| WHSV für $C_4$-Einsatz | 2,94 | 2,83 | 3,0 | 3,05 | 3,05 | 3,05 | 2,94 | 2,17 |
| WHSV für Wasserdampf | 2,33 | 2,33 | 2,33 | 2,09 | 2,05 | 2,03 | 2,0 | 2,0 |
| Isobutan (Gew.-%) | 4,09 | 7,45 | 6,50 | 8,30 | 2,91 | 4,00 | 6,19 | 7,13 |
| Propen (Gew.-%) | 2,98 | 3,17 | 3,22 | 2,91 | 2,98 | 2,85 | 2,61 | 2,37 |
| n-Butan (Gew.-%) | 23,43 | 23,69 | 23,76 | 23,76 | 23,05 | 23,58 | 24,24 | 23,70 |
| n-Buten-1 (Gew.-%) | 15,08 | 13,84 | 14,01 | 14,03 | 15,38 | 15,19 | 15,02 | 14,16 |
| 1-Buten (Gew.-%) | 13,92 | 14,02 | 14,33 | 13,72 | 14,30 | 14,00 | 14,00 | 15,36 |
| t-Buten-2 (Gew.-%) | 19,87 | 18,53 | 18,76 | 18,36 | 20,43 | 20,01 | 18,29 | 18,88 |
| 1-Buten-2 (Gew.-%) | 15,50 | 14,32 | 14,42 | 14,35 | 15,81 | 15,58 | 15,33 | 14,25 |
| $C_{5+}$ (Gew.-%) | 4,99 | 4,82 | 4,97 | 4,42 | 5,00 | 4,66 | 4,29 | 3,99 |
| $\leq C_3$ (Gew.-%) | 0,14 | 0,16 | 0,03 | 0,15 | 0,14 | 0,13 | 0,03 | 0,16 |
| Umwandlung n-Butene (%) | 32,5 | 34,4 | 35,4 | 40,2 | 33,2 | 33,2 | 34,8 | 38,2 |
| Selektivität für Iso-buten (%) | 63,1 | 63,2 | 63,5 | 64,7 | 63,8 | 64,7 | 66,9 | 70,2 |
| Selektivität für Pro-pen (%) | 13,5 | 14,5 | 14,3 | 13,7 | 13,3 | 13,2 | 12,5 | 10,9 |
| Selektivität für $C_{5+}$ (%) | 22,7 | 21,7 | 22,0 | 20,8 | 22,3 | 21,5 | 20,5 | 18,2 |
| Selektivität für $\leq C_3$ (%) | 0,7 | 0,6 | 0,2 | 0,8 | 0,6 | 0,6 | 0,1 | 0,7 |
| RZA für Isobuten | 409,2 | 396,8 | 429,9 | 418,5 | 436,2 | 427,0 | 411,6 | 333,3 |

**Beispiel 4**

37,5 g des nach Beispiel 1 hergestellten und nach Beispiel 2 mit Ammoniumfluorid behandelten Alumosilikats wurden mit 12,5 g des nach Beispiel 3 mit $MgCl_2$-Lösung behandeltem Kaolin vermischt, analog Beispiel 1 verformt und anschlie-ßend bei 500° C 3 Stunden lang calciniert.

Der so hergestellte Katalysator wurde gemäß der in Beispiel 1 beschriebenen Verfahrensweise für die Umwandlung von n-Buten zu iso-Buten eingesetzt. Die dabei erhaltenen Ergebnisse sind in Tabelle 4 zusammengefaßt.

9

## Tabelle 4

| Betriebsstunden | 100 | 200 | 300 | 400 | 500 | 600 | 700 | 800 | 900 | 1000 |
|---|---|---|---|---|---|---|---|---|---|---|
| WHSV für C$_4$-Einsatz | 2,33 | 2,33 | 2,33 | 2,5 | 2,5 | 2,5 | 2,44 | 2,31 | 2,39 | 2,47 |
| WHSV für Wasserdampf | 2,0 | 2,0 | 2,0 | 2,0 | 2,0 | 2,0 | 2,0 | 2,0 | 2,0 | 2,0 |
| Isobutan (Gew.-%) | 4,23 | 8,22 | 8,39 | 6,17 | 5,33 | 4,41 | 3,14 | 8,29 | 8,09 | 7,68 |
| Propen (Gew.-%) | 4,49 | 3,33 | 3,79 | 3,47 | 3,50 | 3,91 | 3,71 | 3,05 | 3,13 | 3,25 |
| n-Butan (Gew.-%) | 23,33 | 23,48 | 23,42 | 23,76 | 23,74 | 23,69 | 23,06 | 23,56 | 23,56 | 23,72 |
| n-Buten-1 (Gew.-%) | 13,06 | 12,75 | 12,58 | 13,23 | 13,39 | 13,45 | 13,89 | 13,10 | 13,11 | 13,15 |
| 1-Buten (Gew.-%) | 18,35 | 17,72 | 17,59 | 18,19 | 18,14 | 18,12 | 18,89 | 17,36 | 17,45 | 17,52 |
| t-Buten-2 (Gew.-%) | 17,20 | 16,80 | 16,54 | 17,23 | 17,43 | 17,56 | 18,12 | 17,40 | 17,12 | 17,16 |
| 1-Buten-2 (Gew.-%) | 13,55 | 13,27 | 13,06 | 13,58 | 13,80 | 13,86 | 14,29 | 13,48 | 13,50 | 13,55 |
| C$_{5+}$ (Gew.-%) | 5,43 | 4,16 | 4,34 | 4,09 | 4,34 | 4,70 | 4,61 | 3,77 | 3,81 | 3,71 |
| Umwandlung n-Butene (%) | 50,17 | 47,86 | 46,51 | 39,66 | 40,07 | 40,60 | 37,44 | 39,91 | 41,70 | 42,64 |
| Selektivität für Iso-buten (%) | 64,11 | 69,57 | 67,61 | 69,92 | 69,11 | 67,05 | 68,67 | 71,10 | 70,85 | 70,83 |
| Selektivität für Pro-pen (%) | 15,67 | 13,06 | 14,56 | 13,33 | 13,32 | 14,46 | 13,49 | 12,46 | 12,69 | 13,15 |
| Selektivität für C$_{5+}$ (%) | 18,96 | 16,32 | 16,69 | 15,71 | 16,54 | 17,37 | 16,76 | 15,39 | 15,47 | 15,0 |
| RZA für Isobuten | 428,2 | 433,2 | 469,9 | 454,9 | 453,5 | 453,0 | 461,8 | 401,07 | 416,8 | 433,2 |

## Beispiel 5

Über den nach Beispiel 2 hergestellten Katalysator wurde reines n-penten geleitet, wobei in einem röhrenförmigen Reaktor mit einer Katalysatormenge von 20 cm bei Atmosphärendruck und bei einer Temperatur von 500°C gearbeitet wurde.

Die Ergebnisse dieses Versuches sind zusammengefaßt Folgende:

WHSV für penten: 2
WHSV für $H_2O$:
Umwandlung, Gew.-%: 25,3
Selektivität i-Pentene: 84,2 %
Selektivität $\leqslant C_5$: 17,3 %
Selektivität $C_6+$: 18,5 %
Zur Darstellung dieser Ergebnisse wurden folgende Definitionen verwendet:

$$\text{Umwandlung \% } = 100 \; - \sum \text{lineare Pentene (Reaktorausgang)}$$

$$\text{Selektivität} \leqslant C_5 = \frac{\text{Gew.-\%} \leqslant C_5 \cdot 100}{\sum \underset{\text{Eingang}}{\text{n-Pentene}} - \sum \underset{\text{Ausgang}}{\text{n-Pentene}}}$$

$$\text{Selektivität} \quad C_6^+ = \frac{\text{Gew.-\%} \; C_{6+} \cdot 100}{\sum \underset{\text{Eingang}}{\text{n-Pentene}} - \sum \underset{\text{Ausgang}}{\text{n-Pentene}}}$$

$\leqslant C_5$ : Kohlenwasserstoffe mit weniger als 5 Kohlenstoffatomen
$C_6+$ : Kohlenwasserstoffe mit 6 und mehr Kohlenstoffatomen

**Beispiel 6** (zum Vergleich)

Ein nach Beispiel 2 hergestellter Katalysator wurde 2 8tun-den bei 700°C calciniert. Über das so erhaltene produkt wurde wie im Beispiel 1 beschrieben $C_4$-Einsatz und Wasserdampf geleitet. Dieses produkt zeigte dabei keine katalytische Aktivität für die Umwandlung von n-Buten zu iso-Buten.

**Patentansprüche**

1) Katalysator, dadurch gekennzeichnet, daß er erhältlich ist, indem man kristallisiertes Alumosilikat, das 0,05 bis 2 Gew.-% Fluor enthält und das ein Molverhältnis von Silicium zu Aluminium von 100:1 bis 10 000:1 aufweist, mit einem Kaolinit enthaltenden Tonmineral mischt, das mit der Lösung eines oder mehrerer Alkali- und/oder Erdalkalisalze vorbehandelt wurde, gegebenenfalls das Gemisch mit Bindemitteln und/oder Füllstoffen versetzt und verformt, und bei 350 bis 550° C calciniert.

2) Verfahren zur Herstellung eines Katalysators, dadurch gekennzeichnet, daß man kristallisiertes Alumosilikat, das 0,05 bis 2 Gew.-% Fluor enthält und das ein Molverhältnis von Silicium zu Aluminium von 100:1 bis 10 000:1 aufweist, mit einem Kaolinit enthaltenden Tonmineral mischt, das mit der Lösung eines oder mehrerer Alkaliund/oder Erdalkalisalze vorbehandelt wurde, gegebenenfalls das Gemisch mit Bindemitteln und/oder Füllstoffen versetzt und verformt, und bei 350 bis 550° C calciniert.

3) Verfahren zur katalytischen Skelettisomerisierung von n-Alkenen zu iso-Alkenen, dadurch gekennzeichnet, daß man die Isomerisierung in Gegenwart von Katalyshtoren durchführt, die erhbitlich sind, indem man kristallisiertes Alumosilikat, das ein Molverhältnis von Silicium zu Aluminium von 100:1 bis 10 000:1 aufweist, mit einem Kaolinit enthaltenden Tonmineral mischt, gegebenenfalls das Gemisch mit Bindemitteln und/oder Füllstoffen versetzt und verformt, und bei 350 bis 550° C calciniert.

4. Verfahren nach Anspruch 3, dadurch gekennzeichnet, daß ein kristallisiertes Alumosilikat tingesetzt wird, das 0,05 bis 2 Gew.-% Pluor enthält.

5. Verfahren nach Anspruch 3 oder 4, dadurch gekennzeichnet, daß ein Kaolinit enthaltendes Tonmineral eingesetzt wird, das mit der Lösung eines oder mehrerer Alkali- und/oder Erdhladlisalze vorbehandelt wurde.

6. Verfahren nach Ansprüchen 3 bis 5, dadurch gekennzeichnet, daß ein Kristallisiertes Alumosilikat der Pentasil-Familie eingesetzt wird.

7. Verfahren nach Ansprüchen 3 bis 6, dadurch gekennzeichnet, daß das Gewichtsverhältnis von kristallisiertem Alumosilikat zu Kholinit enthdltendem Tonmineral im Bereich 0,4:1 bis 20:1 liegt.

8. Verfahren nach Ansprüchen 3 bis 7, dadurch gekennzeichnet, daß als Kaolinit enthaltendes Tonmineral Kaolin bingesetzt wird.

## Claims

1. Catalyst, characterised in that it is obtainable by mixing a crystalline aluminosilicate which contains 0.05 to 2% by weight of fluorine and which has a molar ratio of silicon to aluminium of 100:1 to 10,000:1 with a kaolinite-containing clay mineral which has been pretreated with a solution of one or more alkali metal and/or alkaline earth metal salts, optionally adding binders and/or fillers to the mixture, moulding the latter and calcining the mouldings at 350 to 550° C.

2. Process for the preparation of a catalyst, characterised in that a crystalline aluminosilicate which contains 0.05 to 2% by weight of fluorine and which has a molar ratio of silicon to aluminium of 100:1 to 10,000:1 is mixed with a kaolinite-containing clay mineral which has been pretreated with a solution of one or more alkali metal and/or alkaline earth metal salts, binders and/or fillers are optionally added to the mixture, the latter is moulded and the mouldings are calcinde at 350 to 550°

3. Process for the catalytic skeletal isomerisation of n-alkenes to iso-alkenes, characterised in that the isomerisation is carried out in the presence of catalysts which are obtainable by mixing a crystalline aluminosilicate which has a molar ratio of silicon to aluminium of 100:1 to 10,000:1 with a kaolinite-containing clay mineral, optionally adding binders and/or fillers to the mixture, moulding the latter and calcining the mouldings at 350 to 550°c.

4. Process according to Claim 3, characterised in that a crystalline aluminosilicate which contains 0.05 to 2% by weight of fluorine is employed.

5. Process according to Claim 3 or 4, characterised in that a kaolinite-containing clay mineral which has been pretreated with a solution of one or more alkali metal and/or alkaline earth metal salts is employed.

6. Process according to Claims 3 to 5, characterised in that a crystalline aluminosilicate of the pentasil family is employed.

7. Process according to Claims 3 to 6, characterised in that the weight ratio of the crystalline aluminosilicate to the kaolinite-containing clay mineral i sin the rang from 0.4:1 to 20:1.

8. Process according to Claims 3 to 7, characterised in that kaolin is employed as the kaolinite-containing clay mineral.

## Revendications

1. Catalyseur, caractérisé en ce qu'il peut être obtenu en mélangeant un aluminosilicate cristallisé qui contient 0,05 à 2 % en poids de fluor et qui présente un rapport molaire du silicium à l'aluminium de 100:1 à 10 000:1, avec une ergile minérale comtenant de la kaolinite, qui e été préalablement traitée avec une solution d'un ou plusieurs sels de métaux alcalins et/ou alcalino-terreux, en ajoutant éventuellement eu mélamge des liants et/ou des charges et en lui donnant une forme, et en le celcinemt à 350-550° C.

2. Procédé de production d'un catalyseur, caractérise en ce qu'on méelange un aluminosilicate cristallisé qui contient 0,05 à 2 % en poids de fluor et qui présente un rapport molaire du silicium à l'aluminium de 100:1 à 10 000:1, avec une argile minérale contenant de la keolinite, qui a été préalablement traitée avec une solution d'un ou plusieurs sels de méteux alcalins et/ou alcalino-terreux, on ajoute éventuellement au mélenge des liants et/ou des charges et on lui donne une forme, et on le calcine à 350-550° C.

3. Procédé de transposition moléculaire catalytique de n-alcènes en iso-alcènes, caractérisé en ce qu'om conduit l'isomérisation en présence de catalyseurs qui peuvent être obtenus en mélangeant un aluminosilicate cristallisé, qui présente un rapport molaire du silicium à l'aluminium de 100:1 à 10 000:1, avec une argile minérale contenant de la kaolinite, en ajoutant éventuellement au mélange des lients et/ou des charges et en lui donnant une forme, et en le calcinant à 350-550° C.

4. Procéde suivant la revendication 3, caractérisé en ce qu'on utilise un aluminosilicate cristallisé qui contient 0,05 à 2 % en poids de fluor.

5. Procédé suivant la revendication 3 ou 4, caractérisé en ce qu'on utilise une argile minérale contenant de la kaolinite, qui a été préalablement traitée avec une solution d'un ou plusieurs sels de métaux alcalins et/ou alcalino-tarraux.

6. Procédé suivant les revendications 3 à 5, caractérisé am ca qu'om utilise un aluminosilicate cristallisé da la famille du pentasil.

7. Procédé suivant les revendications 3 à 6, caractérisé en ce que le rapport en poids de l'aluminosilicate cristallisé à l'argile minérale contenant da la kaolinita se situe dans l'intervalle de 0,4:1 à 20:1.

8. Procédé suivant les revendications 3 à 7, caractérisé an ce qu'on utilise du kaolin comme argile minérale contenant de la kaolinite.